**Europäisches Patentamt**

**European Patent Office**

**Office européen des brevets**

(19)

(11) **EP 1 110 560 A2**

(12) **EUROPÄISCHE PATENTANMELDUNG**

(43) Veröffentlichungstag:
   **27.06.2001   Patentblatt 2001/26**

(51) Int Cl.⁷: **A61L 15/12**

(21) Anmeldenummer: **00124803.8**

(22) Anmeldetag: **14.11.2000**

(84) Benannte Vertragsstaaten:
   **AT BE CH CY DE DK ES FI FR GB GR IE IT LI LU MC NL PT SE TR**
   Benannte Erstreckungsstaaten:
   **AL LT LV MK RO SI**

(30) Priorität: **23.12.1999  DE 19962747**

(71) Anmelder: **Beiersdorf Aktiengesellschaft**
   **20245 Hamburg (DE)**

(72) Erfinder:
   • **Andrews, Arthur Hugh**
     **25337 Kölln-Reisiek (DE)**
   • **Bodenschatz, Stefan, Dr.**
     **21614 Buxtehude (DE)**

(54) **Orthopädische Bandage**

(57)   Orthopädische Bandage, enthaltend ein flexibles Trägermaterial und eine Klebemasse, dadurch gekennzeichnet, daß die Klebemasse
wenigstens teilweise ein thermoplastisches Verhalten aufweist,
bei einer Temperatur unterhalb von 40 °C ein Schermodul von mehr als $10^6$ Pa aufweist. im Temperaturbereich zwischen 55 und 65 °C bei einer Frequenz von 100 rad/s ein Verhältnis des viskosen Anteils zum elastischen Anteil von größer 2,5 aufweist und eine Schälfestigkeit zwischen zwei Lagen der Bandage unterhalb einer Temperatur von 40 °C größer 1 N/mm aufweist.

Figur 1

EP 1 110 560 A2

**Beschreibung**

Die Erfindung betrifft eine orthopädische Bandage für medikale Anwendungen zur Stabilisierung und Fixierung von Gelenken und Extremitäten.

Bei Frakturen von Gelenken oder Extremitäten kommt klassischerweise ein Gipsverband zum Zuge, um die Stabilisierung beziehungsweise Fixierung des betroffenen Körperteils zu gewährleisten. Durch den Gipsverband wird die Bewegungsfähigkeit soweit eingeschränkt, daß das Knochengewebe wieder zusammenwachsen kann.

Weiterhin können Gipsverbände auch bei Rupturen von Bändern in Gelenken eingesetzt werden. Auch in diesem Fall soll der Verband jedwede Belastung beziehungsweise Bewegung unterbinden.

Derartige Gipsverbände können aus Gipsbinden oder synthetischen Gipsbinden auf Reaktionsharzbasis bestehen. Bei beiden Systemen erfolgt eine Aushärtung durch das Benetzen mit Wasser. Dadurch wird in Verbindung mit den in den Binden eingearbeiteten Verstärkungsmaterialien die Festigkeit erreicht.

Diese Methoden sind bekannt. Sie haben aber auch Nachteile für den Anwender und Patienten. Natürliche Gipsverbände sind relativ kostengünstig, dafür aber schwer und nur begrenzt haltbar. Synthetische Gipsbinden müssen mit Handschuhen angelegt werden. Das Aushärten der Verbände benötigt bis zu 30 Minuten. Die synthetischen Gipsbinden müssen sehr aufwendig verpackt sein, weil sie im Kontakt mit Luftfeuchte aushärten.

Verletzungen von Bändern in Gelenken kann man auch mit der funktionellen Verbandtechnik, dem sogenannten Taping, entgegen treten. Die Verbandtechnik ist darüber hinaus eine Behandlungsmethode zur Prophylaxe von Verletzungen, Krankheiten und Veränderungen am Bewegungsapparat. Taping hat zum Ziel, die Kapsel-Band-Strukturen gezielt nachzubilden und dadurch eine selektive Unterstützung und Stabilisierung zu erreichen. Eine Immobilisation wird damit jedoch nicht erzielt, wie sie mit Gipsverbänden angestrebt wird.

Der eigentliche Tapeverband wird dabei streifenweise aus vorzugsweise unelastischen selbstklebenden Bändern, sogenannten Zügeln, oder in Verbindung mit kurzzugelastischen selbstklebenden Bändern angelegt. Er schützt, stützt und entlastet gefährdete, geschädigte oder gestörte Anteile einer Funktionseinheit. Er erlaubt die funktionelle Belastung im schmerzfreien Bewegungsraum, verhindert aber extreme oder schmerzhafte Bewegungen.

Als Trägermaterialien haben sich insbesondere Vliese, Gewebe oder Gewirke bewährt, die mit einem druckempfindlichen Kleber beschichtet sind. Auch beim Anlegen mehrerer Lagen bleiben diese Verbände weitgehend flexibel.

Eine zum Ruhigstellen eines Körperteils geeignete Bandage ist in der EP 0 352 095 B beschrieben. Die Bandage besteht aus einem Substrat, dessen Oberflächen mit einer härtbaren flüssigen Verbindung imprägniert sind. Auf den Oberflächen sind weiterhin Abdeckungen vorhanden, die für Wasser durchgängig sind. Bei den Abdeckungen handelt es sich bevorzugt um einen Vlies- oder Gewebeträger, der u.a. eine Fluorverbindung oder ein Silikon enthält.

WO 97/38646 offenbart eine wasservernetzte Bandage auf Silikonbasis. Eine thermoaktivierbare Ausrüstung ist nicht erwähnt.

US 5,412,035 beschreibt ein thermoaktivierbares Klebesystem, welches rekristallisiert. Die notwendige Stabilität und Festigkeit für orthopädische Bandagen sind nicht beschrieben und für den Fachmann aus diesem System nicht abzuleiten.

US 5,387,450 beschreibt ein thermoaktivierbares und thermodeaktivierbares Klebesystem, welches geringe Temperaturübergänge aufweist. Die notwendige Stabilität und Festigkeit für orthopädische Bandagen sind ebenfalls nicht offenbart und für den Fachmann daher nicht offensichtlich.

Aus der DE 37 29 262 A1 ist ein thermoplastisches Konstruktionsmaterial bekannt, das einen Träger und eine Polymerzubereitung enthält, die aus einem schmelzbaren Polymerisat, einer kristallisierenden Carbonsäure oder einem Carbonsäurederivat und gegebenenfalls aus Kristallisationsmodifikatoren besteht. Das thermoplastische Konstruktionsmaterial kann besonders für Stützverbände verwendet werden.

Die EP 0 309 842 A2 offenbart eine Verbund-Stoffverbindung in Form von Folien, aufgewickelten Bändern, oder Platten, die gleichmäßig verteilte Öffnungen mit einer Abmessung von 1 bis 12 mm aufweisen, einen freien Durchgang von mindestens 40 % der gesamten Oberfläche lassen, bei einer Temperatur in dem Temperaturbereich von 35 bis 90 °C formbar sind und auf sich selbst kleben. Diese sind mit einem weitmaschigen Textilsubstrat mit einem spezifischen Gewicht von nicht mehr als 500 g/m$^2$ kombiniert, dessen Körper nacheinander mit mindestens zwei verschiedenen Materialien getränkt oder überzogen und dann erneut getränkt und vollständig umhüllt wird, wobei das erste Material, das sich im Inneren oder auf der Oberfläche des Substrats befindet, von der Familie der Polymere, Copolymere, oder amorphen oder halbkristallinen Legierungen ist, die eine Erweichungstemperatur von nicht mehr als 80°C haben, und jenseits dieser Erweichungstemperatur ein elastoviskoses bis gummiartiges, aber nicht flüssiges Verhalten aufweisen. Das zweite Material, das sich auf der äußeren Oberfläche des ersten Materials befindet, ist von der Familie der Polymere, Copolymere oder halbkristallinen Legierungen, die einen Gehalt an strukturellen Einheiten vom Typ aliphatischer Ester von mindestens 80 % haben, und deren Schmelztemperatur zwischen 35 und 80 °C liegt, und die während einer gewissen Zeit nach der Schmelzung auf sich selbst kleben, so daß sie in zwei oder mehreren Schichten laminiert werden können.

Die DE 196 20 109 A1 beschreibt ein selbstklebend ausgerüstetes Trägermaterial mit einer mindestens auf einer Seite vollflächig aufgebrachten Heißschmelzselbstklebemasse. Die thermoplastische Klebemasse ist geschäumt. Das Produkt weist bei einem Masseauftrag von mindestens 20 g/m$^2$ eine Luftdurchlässigkeit von mindestens 3 cm$^3$/cm$^2$/s auf.

Aufgabe der Erfindung ist es, eine Bandage zur Verfügung zu stellen, die aufgrund ihrer Ausgestaltung, ihres Materials und ihrer Eigenschaften zur Stabilisierung und Fixierung von Gelenken und Extremitäten geeignet ist.

Gelöst wird diese Aufgabe durch eine Bandage, wie sie im Hauptanspruch dargelegt ist. Gegenstand der Unteransprüche sind dabei vorteilhafte Weiterbildungen der Bandage.

Demgemäß betrifft die Erfindung eine orthopädische Bandage, die ein flexibles Trägermaterial und eine Klebemasse enthält, wobei die Klebemasse

> a) wenigstens teilweise ein thermoplastisches Verhalten aufweist,
>
> b) bei einer Temperatur unterhalb von 40 °C ein Schermodul von mehr als 10$^6$ Pa aufweist,
>
> c) im Temperaturbereich zwischen 55 und 65 °C bei einer Frequenz von 100 rad/s ein Verhältnis des viskosen Anteils zum elastischen Anteil von größer 2,5 aufweist und
>
> d) eine Schälfestigkeit zwischen zwei Lagen der Bandage unterhalb einer Temperatur von 40 °C größer 1 N/mm aufweist.

Die Klebmasse kann sich aus thermoplastischen und aus nichtthermoplastischen Anteilen zusammensetzen, also insgesamt ein thermoplastisches Verhalten aufzeigen. Dies gilt insbesondere, wenn die Klebmasse nur aus thermoplastischen Anteilen besteht.

Bevorzugterweise liegt die Aktivierungstemperatur der Klebmasse zwischen 30 °C und 70 °C.

Weiter bevorzugt ist die orthopädische Bandage nach dem Unterschreiten der Aktivierungstemperatur nicht klebrig, also insbesondere in einem Temperaturbereich von -25 °C bis 30 °C.

Die polymeren Verbindungen in der Klebmasse basieren in einer bevorzugten Ausführungsform wenigstens zum Teil auf Urethan-, Epoxid-, Harnstoff-, Melamine-, Polyamid-, Polyester-, Polyether-, gesättigten oder ungesättigten Polyolefin-, Polyvinyl-, Sulfonat-, Acrylat- oder Methacrylatverbindungen und/oder deren Mischungen.

Besonders bevorzugt werden polymere Verbindungen in der Klebemasse, die Hydroxylpolyesterpolyurethane oder Hydroxylpolyetherpolyurethane und/oder deren Mischungen beinhalten.

Generell kann ein Zusatz von klebrigmachenden Harzen, Plastifizierungsmittel, Verdicker, Weichmachern, Additiven, Füllstoffen, Pigmenten, Fasern, Stabilisatoren und/oder Wirkstoffen zu vorteilhaften Ausrüstungen führen.

Der Anteil der polymeren Verbindung in der Klebemasse beträgt mindestens 1 Gewichtsprozent. Je nach Anwendung kann der Anteil der polymeren Verbindung zwischen 1 Gew.-% und 100 Gew.-% variieren.

Schließlich hat es sich als vorteilhaft herausgestellt, wenn die Klebemasse wenigstens teilweise nachträglich chemisch und/oder physikalisch vernetzt worden ist.

Der Erweichungspunkt der Klebemasse beträgt mindestens 40 °C. Vorteilhaft sind für dauerhafte Anwendungen Erweichungspunkte von 50 °C bis 140 °C, besonders vorteilhaft 55 °C bis 90 °C.

Weiter weisen die erfindungsgemäßen Klebemassen eine Aktivierungstemperatur auf. Oberhalb der Aktivierungstemperatur wird die Klebemasse klebrig. In einer speziellen Ausführung ist die Aktivierungstemperatur kleiner als die Temperatur am Erweichungspunkt.

Je nach Rezeptierung der Klebemasse kann diese von sehr stark selbstklebrig bis stark selbstklebrig eingestellt werden. Je stärker die Klebrigkeit ist, desto besser ist die Stabilität der Bandage direkt nach der Applikation und die Sicherheit für die Therapie.

Die Klebrigkeit hängt von der Art der polymeren Verbindung in der Klebmasse, dem Molekulargewicht, der Molekulargewichtsverteilung, den weiteren Komponenten und dem Herstellungsprozeß u.a. ab.

Die Produkteigenschaften wie Anfaßklebrigkeit und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur der Klebmasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G" viskoser Anteil) und dem Speichermodul (G' elastischer Anteil) ermittelt. Für das subjektive Empfinden der Anfaßklebrigkeit (Tack) wird eine hohe Frequenz gewählt, sowie für die Scherfestigkeit eine niedrige Frequenz. Eine hoher Zahlenwert bedeutet eine bessere Anfaßklebrigkeit und eine schlechtere Scherstabilität.

$$Q = \tan \delta = G''/G'$$

Bevorzugt werden erfindungsgemäß thermoaktivierbare Klebmassen, bei denen das Verhältnis des viskosen Anteils zum elastischen Anteil bei einer Temperatur von 55 °C bis 65 °C und bei einer Frequenz von 100 rad/s größer 2,5 ist.

Nach dem Unterschreiten der Aktivierungstemperatur ist die Klebemasse sehr schwach bis nicht klebrig. Diese Eigenschaft ermöglicht somit, daß die Bandage nicht mit der Kleidung oder anderen Gegenständen verklebt.

Vor der Applikation der Bandage wird die Bandage über die Aktivierungstemperatur der Klebemasse erhitzt und somit in einen klebrigen Zustand versetzt. Eine gute Festigkeit und strukturelle Stabilität des Stützverbandes wird durch Applikation von mehreren Lagen der Bandage übereinander hergestellt.

Nach der Applikation der Bandage erhöht sich die Festigkeit und strukturelle Stabilität durch kristalline Strukturen, die sich unterhalb der Aktivierungstemperatur der Klebemasse ausbilden. Die Bildung solcher kristallinen Strukturen erfolgt innerhalb von weniger als 2 h nach der Abkühlung auf eine Temperatur unterhalb der Aktivierungstemperatur.

Vorteilhaft ist die Bildung solcher kristallinen Strukturen der Klebemasse beziehungsweise der Klebemasse verwendeten polymeren Verbindung nach 30 s bis 30 min, besonders bevorzugt nach 2 min bis 5 min. In dieser Zeit gewinnt die Bandage an Stabilität.

Die Schälfestigkeit zwischen zwei Lagen der Bandage ist unterhalb einer Temperatur von 40 °C größer 1 N/mm. Bevorzugt wird, wenn diese Schälfestigkeit größer 2,5 N/mm ist, ganz besonders bevorzugt größer 5 N/mm.

In einer besonderen Ausführung der erfindungsgemäßen Bandage kann ein Nachmodellieren nach Anlegen an ein Körperteil durch erneutes zumindest lokales Erwärmen über die Aktivierungstemperatur der Klebemasse erfolgen.

Nach Erreichung der vollständigen Stabilität, also bei einer Temperatur von unter 40 °C, beträgt das Schermodul der Klebemasse wenigstens 1.000.000 Pa, bevorzugt 1.000.000 Pa bis 600.000.000 Pa.

Die Bandage ist insbesondere mit mindestens 10 g/m$^2$ Klebemasse beschichtet. Bevorzugt werden 40 bis 1000g/m$^2$, besonders bevorzugt 55 g/m$^2$ bis 800 g/m$^2$, eingesetzt.

Eine günstige Ausführungsform der Klebemasse weist eine Dichte von weniger als 2000 kg/m$^3$, bevorzugt 300 kg/m$^3$ bis 1500 kg/m$^3$, auf.

Die Klebemasse kann als Lösung, Dispersion, Emulsion oder als 100 %-Systeme oder deren Kombinationen eingesetzt werden, wobei die Wahl des geeigneten Beschichtungsverfahrens von den Eigenschaften der Klebemasse abhängig ist. Als Beschichtungsverfahren sind die üblichen Rakel-, Tränk-, Druck-, Sprüh-, Spinn-; Imprägnier- und Transferverfahren zu nennen. Besonders vorteilhaft ist, wenn die Klebemasse das flexible Trägermaterial zumindest teilweise durchdringt oder imprägniert, also zumindest in Teilbereichen in das Trägermaterial beziehungsweise zwischen die einzelnen Fasern oder Lagen des Trägermaterials einsinkt.

Vorteilhaft, besonders aus ökologischen Gesichtspunkten, ist die Verwendung von 100 %-Systemen, da bei ihnen verfahrenstechnisch ein Entfernen der Trägermatrix, d.h. der Hilfsmittel, vermieden wird, wodurch sich die Verarbeitungsproduktivität steigert und sich gleichzeitig der Maschinen- und Energieaufwand reduziert.

In einer bevorzugten Ausführungsform, insbesondere für die Verwendung bei medizinischen Produkten, wird die Klebemasse partiell auf dem Trägermaterial aufgetragen, beispielsweise durch Rasterdruck, Thermosiebdruck oder Tiefdruck.

Bevorzugt wird der Auftrag der Klebemasse auf das Trägermaterial in Form von polygeometrischen Kalotten, Körpern oder Segmenten.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Die angelegten Abführungskanäle ermöglichen ein Ableiten der Feuchtigkeit auch unter Verwendung eines mehrlagigen Verbandes.

Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Ferner kann die Klebemasse beispielsweise auch aufgesprüht sein, was ein mehr oder weniger unregelmäßiges Auftragsbild ergibt.

Die Klebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des partiellen Auftrages soll, ohne die Erfindung einzuschränken, anhand des Thermosiebdrucks erläutert werden. Dieser besteht in der Verwendung einer rotierenden beheizten, nahtlosen, trommelförmigen perforierten Rundschablone, die über eine Düse mit der thermoplastischen Ausrüstung beschickt wird. Eine speziell geformte Düsenlippe (Rund- oder Vierkantrakel) preßt die über einen Kanal eingespeiste thermoplastische Ausrüstung durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der sich dabei ergebenden kleinen Ausrüstungskalotten geschieht dabei nach folgendem Mechanismus:

Der Düsenrakeldruck fördert die thermoplastische Ausrüstung durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Klebemasse und die innere Kohäsion des Hotmelts wird von der auf dem Träger bereits haftenden Basis der Kalotten der in den Löchern begrenzte Vorrat an Haftschmelzklebemasse konturenscharf abgezogen bzw. durch den Rakeldruck auf den Träger gefördert.

Nach Beendigung dieses Transportes formt sich, abhängig von der Rheologie der Klebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Klebemasse ab.

Bei einer besonderen Einstellung befindet sich zumindest bei einem Teil der geometrischen Kalotten, Körper und/oder Segmente die Basisfläche, mit der diese auf dem Trägermaterial haften, innerhalb der Projektionsfläche der geometrischen Kalotten, Körper und/oder Segmente, die sich daraus ergibt, daß die geometrischen Kalotten, Körper und/oder Segmente lotrecht auf das Trägermaterial projiziert werden.

Der Flächendeckungsgrad der Beschichtung sollte mindestens 20%, bevorzugt 50% bis 100% betragen. Bei einer besonders bevorzugten Ausführung sollte der Flächendeckungsgrad zwischen 75% und 95% betragen.

Es ist somit möglich, durch Auswahl von gezielten Einstellungen beim Herstellprozeß ein luft- und wasserdampfdurchlässiges beschichtetes Flächengebilde zu erzielen. Die Luftdurchlässigkeit beträgt in diesem Fall mindestens 1 cm/(cm$^{2*}$s). In einer vorteilhaften Ausführung beträgt sie 15 bis 70 cm/(cm$^{2*}$s) und darüber. Die Wasserdampfdurchlässigkeit kann somit bei einem Wert von größer 500 g/(m$^{2*}$24h) eingestellt werden. Spezielle Einstellungen erlauben eine Wasserdampfdurchlässigkeit von mehr als 1000 und 2000 g/(m$^{2*}$24h).

Für besondere Anwendungen ist es weiterhin vorteilhaft, wenn die Klebemasse mit Inertgasen geschäumt wurde.

Das Schäumen von Dispersionen und Hotmelts zählt zum bekannten Stande der Technik.

Grundsätzlich ist es möglich, Schäume auf physikalischem Weg, auf chemischem Weg oder durch Kombination der beiden Wege zu erzeugen.

Die Klebemassen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind.

Durch den Einsatz von atmungsaktiven Beschichtungen in Verbindung mit elastischen ebenfalls atmungsaktiven Trägermaterialien ergibt sich ein vom Anwender subjektiv angenehm empfundener Tragekomfort.

Ein besonders geeignetes Verfahren zur Herstellung der geschäumten Klebemasse arbeitet nach dem Schaum-Mix-System. Hierbei wird die thermoplastische Klebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (10 Vol.-% bis 80 Vol.-%) in einem Stator/Rotorsystem umgesetzt.

Während der Gasvordruck größer 100 bar ist, betragen die Mischdrucke Gas/Thermoplast im System 40 bis 100 bar, bevorzugt 40 bis 70 bar. Der so hergestellte Klebeschaum kann anschließend über eine Leitung in das Auftragswerk gelangen. Bei dem Auftragswerk finden handelsübliche Düsen, Extruder- oder Kammersysteme Verwendung.

Als weiteres Verfahren zur offenporigen Beschichtung luft- und wasserdampfdurchlässiger Klebeschichten kann das Melt-spin-Verfahren oder Dura-fiber-Verfahren herangezogen werden. Die vliesartig versponnene, über eine beliebig von der Beschichtungsbreite abhängende Anreihung von Düsen kontaktlos aufgetragene Schicht verfügt wie der Schaumauftrag über eine im Vergleich zu vollflächigen Beschichtungen vielfach höhere freie Oberfläche.

Durch die Schäumung der Klebemasse und die dadurch entstandenen offenen Poren in der Masse sind bei Verwendung eines an sich porösen Trägers die mit der Klebemasse beschichteten Produkte gut wasserdampf- und luftdurchlässig. Die benötigte Ausrüstungsmenge wird erheblich reduziert ohne Beeinträchtigung der Klebeigenschaften. Die Klebemasse weist eine überraschend gute Anfaßklebrigkeit auf, da pro Gramm Masse mehr Volumen und damit Oberfläche zum Benetzen des zu beklebenden Untergrundes zur Verfügung steht und die Plastizität der Klebemasse durch die Schaumstruktur erhöht ist, dieses unterstützt die Verbesserung der Anformbarkeit. Auch die Verankerung auf dem Trägermaterial wird dadurch verbessert. Außerdem verleiht die geschäumte Beschichtung, wie bereits oben erwähnt, den Produkten ein weiches und anschmiegsames Anfühlen.

Durch das Schäumen wird zudem die Viskosität der Klebemasse in der Regel gesenkt. Hierdurch wird die Schmelzenergie erniedrigt, und es können auch thermoinstabile Trägermaterialien direkt beschichtet werden.

Als Trägermaterialien sind bereits zahlreiche Materialien auf Folien-, Gewebe-, Gewirke-, Vlies-, Gel-, Laminate oder Schaumstoffbasis vorbeschrieben und werden auch in der Praxis eingesetzt.

Das bevorzugt flexible Trägermaterial kann vor- und/oder nachbehandelt worden sein.

Des weiteren kann die erfindungsgemäße Bandage zu einer endlosen Rolle in Form einer archimedischen Spirale gewickelt werden.

Die orthopädische Bandage wird vorzugsweise mit einem Trennmedium eingedeckt und/oder mit einer Wundauflage oder Polsterung versehen.

Dann kann die orthopädische Bandage sterilisierbar, bevorzugt γ (gamma)-sterilisierbar, sein und die Bandage kann wenigstens teilweise für Röntgenstrahlen durchlässig sein.

Im folgenden soll die erfindungsgemäße orthopädische Bandage anhand von Beispielen beschrieben werden, ohne damit die Erfindung unnötig einschränken zu wollen.

## Beispiel 1

Ein flexibles Gewirk wurde in einer 50%-Polymerdispersion mehrfach getränkt. Als Polymer wurde ein lineares aliphatisches thermoplastisches Polyurethansystem (Dispercoll U 53, Firma Bayer) verwendet, welches unter Wassereinwirkung nicht vernetzt. Die Viskosität der Dispersion betrug ca. 650 mPa*s. Die Einwirkzeit betrug je Tauchgang ca. 2 min. Zwischen den Tränkungen wurden die Hilfsmittel verdampft. Das Schermodul der Klebemasse betrug $3*10^7$ (30.000.000) Pa.

Der Masseauftrag der Klebemasse betrug ca. 120 g/m$^2$. Die fertig ausgerüstete Bandage ist bei Raumtemperatur nicht klebrig. Die Bandage wurde auf 75 °C erwärmt. Eine mehrlagige Anwendung bot eine ausreichende Stabilisierung des Ellenbogengelenkes. Die Bandage konnte mehrere Minuten gut anmodelliert werden, bevor die Rekristallisation zur gewünschten Stabilisierung führte.

## Beispiel 2

Eine längselastische idealbinde mit einer Hotmelt-Ausrüstung partiell beschichtet. Es wurde ein Hydroxyl-Polyesterpolyurethansystem (Dismocoll, Firma Bayer) verwendet. Die Ausrüstung fand bei ca. 130°C statt. Der Masseauftrag betrug 140 g/m$^2$. Das Schermodul der Klebemasse betrug $2*10^7$ (20.000.000) Pa.

Die Bandage wurde auf 70 °C zur Anwendung in einem Umluftofen erwärmt. Ein mehrlagiger Verband konnte mehrere Minuten gut anmodelliert werden. Nach 15 min erreichte die Klebemasse eine Shore A-Härte (DIN 53505) von ca. 80. Der mehrlagige Verband wies eine Luftdurchlässigkeit von 22 cm$^3$/(cm$^{2*}$s) und eine Wasserdampfdurchlässigkeit von mehr als 1000g/(m$^{2*}$24h) auf.

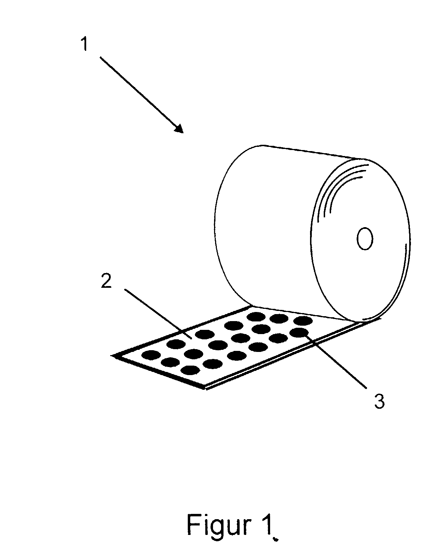

In der Figur 1 ist die gemäß Beispiel 2 hergestellte Bandage 1 gezeigt, die sich aus einem flexiblen Träger 2 und einer auf diesem partiell aufgetragenen selbstklebenden Beschichtung 3 zusammensetzt. Die Bandage 1 ist dabei zu einer endlosen Rolle in Form einer archimedischen Spirale gewickelt.

## Patentansprüche

1. Orthopädische Bandage, enthaltend ein flexibles Trägermaterial und eine Klebemasse, dadurch gekennzeichnet, daß die Klebemasse wenigstens teilweise ein thermoplastisches Verhalten aufweist,

   bei einer Temperatur unterhalb von 40°C ein Schermodul von mehr als $10^6$ Pa aufweist, im Temperaturbereich zwischen 55 und 65 °C bei einer Frequenz von 100 rad/s ein Verhältnis des viskosen Anteils zum elastischen Anteil von größer 2,5 aufweist und eine Schälfestigkeit zwischen zwei Lagen der Bandage unterhalb einer Temperatur von 40 °C größer 1 N/mm aufweist.

2. Orthopädische Bandage gemäß Anspruch 1, dadurch gekennzeichnet, daß die Aktivierungstemperatur der Klebmasse zwischen 30 °C und 70 °C liegt.

3. Orthopädische Bandage gemäß mindestens einem der vorangegangenen Ansprüche, dadurch gekennzeichnet, daß die Klebmasse nach dem Unterschreiten der Aktivierungstemperatur nicht klebrig ist.

4. Orthopädische Bandage gemäß mindestens einem der vorangegangenen Anspruch, dadurch gekennzeichnet, daß die Klebmasse innerhalb 30 s bis 30 min, bevorzugt 2 min bis 5 min, kristalline Strukturen bildet.

5. Orthopädische Bandage gemäß mindestens einem der vorangegangenen Ansprüche, dadurch gekennzeichnet, daß die Klebmasse wenigstens zu einem Teil Urethan-, Epoxid-, Harnstoff-, Melamine-, Polyamid-, Polyester-, Polyether-, gesättigten oder ungesättigten Polyolefin-, Polyvinyl-, Sulfonat-, Acrylat- oder Methacrylatverbindungen und/oder deren Mischungen enthält.

6. Orthopädische Bandage gemäß mindestens einem der vorangegangenen Ansprüche, dadurch gekennzeichnet, daß die Klebmasse wenigstens ein Hydroxylpolyesterpolyurethan oder Hydroxylpolyetherpolyurethan und/oder deren Mischungen beinhaltet.

7. Orthopädische Bandage gemäß mindestens einem der vorangegangenen Ansprüche, dadurch gekennzeichnet, daß die Klebemasse mit einem inerten Gas geschäumt worden ist.

8. Orthopädische Bandage gemäß mindestens einem der vorangegangenen Ansprüche, dadurch gekennzeichnet, daß die Klebemasse das flexible Trägermaterial zumindest teilweise durchdringt oder imprägniert.

9. Orthopädische Bandage gemäß mindestens einem der vorangegangenen Ansprüche, dadurch gekennzeichnet, daß die orthopädische Bandage zu einer endlosen Rolle in Form einer archimedischen Spirale gewickelt ist.

10. Orthopädische Bandage gemäß mindestens einem der vorangegangenen Ansprüche, dadurch gekennzeichnet, daß die orthopädische Bandage mit einem Trennmedium eingedeckt und/oder mit einer Wundauflage oder Polsterung versehen wird.

11. Orthopädische Bandage gemäß mindestens einem der vorangegangenen Ansprüche, dadurch gekennzeichnet, daß die orthopädische Bandage sterilisierbar, bevorzugt $\gamma$ (gamma)-sterilisierbar, ist.

12. Orthopädische Bandage gemäß mindestens einem der vorangegangenen Ansprüche, dadurch gekennzeichnet, daß die orthopädische Bandage wenigstens teilweise für Röntgenstrahlen durchlässig ist.

13. Orthopädische Bandage gemäß mindestens einem vorangegangenen Ansprüche, dadurch gekennzeichnet, daß die orthopädische Bandage nach dem Anlegen an ein Körperteil durch Erwärmen auf eine Temperatur oberhalb der Aktivierungstemperatur nachgeformt werden kann.

1

2

3

Figur 1